# EUROPEAN PATENT APPLICATION

(11) **EP 0 832 897 A2**
(43) Date of publication of application: **01.04.1998**
(21) Application number: 97116329.0
(22) Date of filing: 19.09.1997
(51) Int. Cl.: C07H 1/08, C12N 15/10

(54) **Hydrated zirconium silicate composition for purification of nucleic acids**

(30) Priority: 25.09.1996 US 719603
(71) Applicant: Becton, Dickinson and Company, Franklin Lakes, New Jersey 07417-1880 (US)
(72) Inventor: Woodard, Daniel L., Raleigh, North Carolina 27613 (US)
(74) Representative: Gerbino, Angelo

(57) **Abstract**

The present invention relates to a unique hydrated zirconium silicate composition which binds nucleic acids. The composition is useful in processes for purifying nucleic acid from a sample.

## Description

### BACKGROUND OF THE INVENTION

The present invention relates generally to purification of nucleic acids by solid phase extraction, and more specifically to hydrated zirconium silicate surfaces which are capable of binding and eluting nucleic acids under suitable conditions.

The preparation and purification of high-purity double-stranded (ds) plasmid DNA, single-stranded (ss) phage DNA, chromosomal DNA, agarose gel-purified DNA fragments and RNA is of critical importance in molecular biology. Ideally, a method for purifying nucleic acids should be simple, rapid and require little, if any, additional sample manipulation. Nucleic acids rendered by such a method should be immediately amenable to transformation, restriction analysis, litigation or sequencing. A method with all of these features would be extremely attractive in the automation of nucleic acid sample preparation, a goal of research and diagnostic laboratories.

Typically, the preparation of plasmid DNA from crude alcohol precipitates is laborious, most often utilizing CsCl gradients, gel filtration, ion exchange chromatography, or RNase, proteinase K and repeated alcohol precipitation steps. These methods also require considerable downstream sample preparation to remove CsCl and other salts, ethidium bromide and alcohol. Similar arguments extend when using any of these methods for purifying DNA fragments. A further problem with these methods is that small, negatively-charged cellular components can co-purify with the DNA. Thus, the DNA can have an undesirable level of contamination.

Nucleic acids can also be purified using solid phases. Conventional solid phase extraction techniques have utilized surfaces which either (1) fail to attract and hold sufficient quantities of nucleic acid molecules because of surface design to permit easy recovery of the nucleic acid molecules during elution, or (2) excessively adhere nucleic acid molecules to the surface, thereby hindering recovery of the nucleic acid molecules during elution. Conventional metal surfaces which cause these problems when utilized in solid phase extraction include silica surfaces such as glass and Celite. Adequate binding of nucleic acids to these types of surfaces can be achieved only by utilizing high concentrations of chaotropes or alcohols which are generally toxic, caustic, and/or expensive. For example, it is known that DNA will bind to crushed glass powders and to glass fiber filters in the presence of chaotropes. The chaotropic ions typically are washed away with alcohol, and the DNAs are eluted with low-salt solutions or water. Importantly, RNA and protein do not bind. However, a serious drawback in the use of crushed glass powder is that its binding capacity is low. In addition, glass powders often suffer from inconsistent recovery, incompatibility with borate buffers and a tendency to nick large DNAs. Similarly, glass fiber filters provide a nonporous surface with low DNA binding capacity. Other silicas, such as silica gel and glass beads, are not suitable for DNA binding and recovery. Currently, the solid phase of choice for solid phase extraction of DNA is Celite such as found in Prep-A-Gene™ by Bio-Rad Laboratories. As with the crushed glass powders, high concentrations of chaotropes are required for adequate binding of the DNA to the Celite.

There are numerous protocols for purifying DNA. For example, U.S. Patent 4,923,978 discloses a process for purifying DNA in which a solution of protein and DNA is passed over a hydroxylated support and the protein is bound and the DNA is eluted. U.S. Patent 4,935,342 discloses purification of DNA by selective binding of DNA to anion exchangers and subsequent elution. U.S. Patent 4,946,952 discloses DNA isolation by precipitation with water-soluble ketones. A DNA purification procedure using chaotropes and dialyzed DNA is disclosed in U.S. Patent 4,900,677.

Diatoms have also been utilized for purification of nucleic acids as evidenced by U.S. Patent No. 5,234,809 to Boom et al. and U.S. Patent No. 5,075,430 to Little et al.

### SUMMARY OF THE INVENTION

In order to provide a more effective and efficient technique for the purification of nucleic acids, the present invention relates to a hydrated zirconium silicate composition prepared by refluxing zirconium silicate with sodium hydroxide. This hydrated zirconium silicate composition is particularly useful for purification of nucleic acids from other cellular components.

Such a nucleic acid purification process involves exposure of the hydrated zirconium silicate composition to a sample containing cellular components and then separating the composition from the sample. Nucleic acids in the sample are bound to the hydrated zirconium silicate composition, and can be recovered by elution with a suitable elution buffer.

When used in nucleic acid purification processes, the hydrated zirconium silicate composition of the present invention may be in the form of particles or beads for ease of packaging in a kit format.

### DETAILED DESCRIPTION OF THE INVENTION

The present invention relates to a unique composition of matter. The composition of matter is a hydrated zirconium silicate composition which binds nucleic acids.

The hydrated zirconium silicate composition of the present invention is produced by refluxing zirconium silicate with a suitable alkaline substance such as sodium hydroxide. Suitable zirconium silicate for use in the refluxing reaction is commercially available from Cole-Palmer in a particulate or pelleted form. However, any form or shape of zirconium silicate is suitable for refluxation to produce the hydrated zirconium silicate composition of the present invention.

The refluxing of the zirconium silicate is with a suitable alkaline substance. Such a substance is a composition that contributes to the resultant hydrated zirconium silicate of the present invention being sufficiently electropositive to bind nucleic acids which are relatively electronegative. Most strong bases in water will be suitable alkaline substances for refluxing with zirconium silicate to yield the hydrated zirconium silicate composition of the present invention. Examples of such suitable alkaline substances include sodium hydroxide, potassium hydroxide or any other base which generates a hydroxide ion.

Subsequent reactions to which the bound nucleic acid may be subjected may determine which alkaline substance is preferred. For example, because sodium ions are believed to inhibit Strand Displacement Amplification ("SDA") reactions, a hydroxide ion donor other than sodium hydroxide, such as potassium hydroxide, may be preferred if the nucleic acids bound to the hydrated zirconium silicate composition are to be amplified by SDA.

The amount of alkaline substance used in the refluxing reaction is generally about 0.1M equivalents to about 5M equivalents of the amount of zirconium silicate used in the reaction.

The refluxing reaction used to produce the hydrated zirconium silicate of the present invention is generally conducted for about 18 to about 96 hours at about 100°C (*i*.*e*. reflux). Following such refluxation, the resultant hydrated zirconium silicate composition may optionally be filtered and washed with aqueous acid such as sulfuric acid or hydrochloric acid. An alternative option is to use such aqueous acids to acidify the refluxed suspension of zirconium silicate to a pH of 6.0 or lower, and then filter and wash the hydrated zirconium silicate composition with a suitable wash buffer such as water and/or acetone.

The hydrated zirconium silicate composition produced by the above-described reflux reaction binds nucleic acid. It is believed that the binding of nucleic acid is due at least in part to the attraction of the electropositive hydrated zirconium silicate composition for electronegative nucleic acids. More specifically, the positively charged atoms, for instance Si⁺ from SiO₂ and/or Zr⁺ provide sufficient electropositivity to the hydrated zirconium silicate composition to interact with the negatively charged phosphates of the nucleic acids, thus causing binding.

As set forth in greater detail in the Examples below, the ability of the hydrated zirconium silicate composition of the present invention to bind nucleic acids is quantifiable by the percentage of nucleic acid bound to the surface from an otherwise clean sample (*i*.*e*. without the non-target nucleic acids) under certain conditions. Due to the generally small volumes of samples from which nucleic acids are purified, the percentage of bound nucleic acid is based on a small amount of hydrated zirconium silicate composition (*i*.*e*. 2.5mg), and a total amount of nucleic acid which is generally found in a typical sample from which nucleic acid is purified (*i*.*e*. about 10⁶ target nucleic acid molecules).

Thus, in order to have some utility in a process of purifying nucleic acid from a sample, a nucleic acid binding composition present in an amount of about 2.5mg in a sample containing about 10⁶ target nucleic acid copies should bind at least about 85 percent of the target nucleic acid copies. As shown in the Examples below, the hydrated zirconium silicate composition of the present invention meets this requirement.

The nucleic acids which are bound by the hydrated zirconium silicate composition of the present invention include DNA and RNA obtained from any source, including but not limited to crude cell extracts, biological fluids, phage supernatants, agarose gels and radiolabelling reactions. The nucleic acid, particularly DNA can be double-stranded, single-stranded, circular or linear, and can be variable in size. Conventional techniques for obtaining nucleic acid from any source, well known in the art, are utilized to prepare the nucleic acid for purification. Typical procedures for obtaining nucleic acid end with a suspension of the nucleic acid in solution. For isolation of nucleic acid from biological samples, see, e.g., Harding, J.D. et al., Nucleic Acids Research 17:6947 (1989) and Marko, M.A. et al., Analytical Biochemistry 121:382 (1982). Procedures for isolation of plasmid DNA can be found in Lutze, L.H. et al., Nucleic Acids Research 20:6150 (1990). Techniques for extraction of double-stranded DNA from biological samples can be found in Yamada, O. et al., Journal of Virological Methods 27:203 (1990). Most DNA solutions comprise the DNA in a suitable buffer such as TE (Tris-EDTA), TEA (409 mm Tris-acetate, 1 mm EDTA) buffer, or a lysate. See also Sambrook, J. et al., Molecular Cloning: A Laboratory Manual, 2nd Ed., Cold Spring Harbor Laboratory Press, New York (1989).

Once the nucleic acid is obtained in a suitable sample form, generally suspension or solution, the hydrated zirconium silicate composition of the present invention is exposed to the sample for a period of time sufficient for nucleic acid in the sample to bind to the hydrated zirconium silicate composition. Generally, such exposure is for a time period of about 30 seconds to about 60 minutes at a temperature of about 0°C to about 25°C. A suitable binding buffer, such as, for example, water and acidic buffers with pHs below about 7.0, may be used to assist the binding of nucleic acid to the hydrated zirconium silicate composition.

The hydrated zirconium silicate composition with nucleic acid bound thereto is then separated from the sample. Separation is generally accomplished by centrifugation or filtration.

The hydrated zirconium silicate composition may then be washed, and the nucleic acid may be recovered from the composition. Washing is generally performed with low molecular weight alcohols or other suitable washing solutions such as 80/20 ethanol/50mM Tris, pH 7.0, water or any low pH buffer..

The recovery of bound nucleic acid from hydrated zirconium silicate composition is generally conducted by elution with a suitable elution buffer. Suitable elution buffers include most basic buffers with pHs above about 7.0 such as, for example, any low salt buffer such as 50mM Tris, KPI and KPDG. Preferably, the elution buffer will have minimal interference with processes to which the recovered nucleic acid may be subjected and have minimal negative environmental effects. Typically, elution recovery of nucleic acids from hydrated zirconium silicate composition is conducted over a time period of about one minute to about 60 minutes at a temperature from about 4°C to about 95°C. One way in which such elution may be conducted is to flow the elution buffer past the hydrated zirconium silicate composition with nucleic acid bound thereto to cause the elution of nucleic acid. Alternatively, the hydrated zirconium silicate composition with nucleic acid bound thereto may be placed into the elution buffer, and then separated from the elution buffer in order that the nucleic acid may be recovered from the elution buffer.

The nucleic acid obtained by purification with the hydrated zirconium silicate composition of the present invention may be used without further manipulation for restriction enzyme digestion, cloning, sequencing, diagnostics and the like. The high quality of nucleic acid prepared with the present invention and the speed with which nucleic acid is purified with minimal downstream processing mean that the hydrated zirconium silicate composition can be useful in the automation of nucleic acid sample preparation.

One particularly useful form for the hydrated zirconium silicate composition of the present invention for automated nucleic acid sample preparation is as particles. Particulation of the hydrated zirconium silicate composition provides maximal surface area for binding of nucleic acid. The particles may be of various shapes, including for example, spheres, cubes, oval, capsule-shaped, tablet-shaped, non-descript random shaped, etc., and may be of uniform shape or non-uniform shapes. Whatever the shape of a particle, its diameter at its widest point is generally in the range of from about 0.1 mm to about 0.15 mm. Pellets or beads of a generally spherical shape are the preferred particulate form of the hydrated zirconium silicate composition of the present invention.

The hydrated zirconium silicate composition of the present invention is also particularly useful in a particle form because of its density range of about 2.0mg/uL to about 4.0mg/uL with a preferred value of about 3.3mg/uL. For comparison, hydrated Celite has a density of about 0.9mg/uL. This unusually high density renders it easier to separate the particles of hydrated zirconium silicate with bound nucleic acid from the sample by centrifugation. Also, due to the density of the particles, the pellet formed by the centrifugation is tighter than with less dense compounds allowing a longer time, if desired, between centrifugation and supernatant (sample) removal, as well as an ability to remove more of the supernatant (sample) without dislodging portions of the pellet. The ability to remove more of the supernatant is particularly important as more of the cellular debris and binding buffer are removed, both of which may inhibit or interfere with subsequent processing of the purified nucleic acids.

Particulate forms of the hydrated zirconium silicate composition of the present invention are also advantageous due to their ease of use in kits. Particles can be easily and efficiently packaged in any suitable container as a kit or part of a kit for purification of nucleic acids from samples. Suitable containers for packaging particles of the hydrated zirconium silicate composition of the present invention include vials, tubes, evacuated tubes or containers, and other containers to which a sample containing nucleic acids may be added. Such kits may also include other containers of binding buffer, wash buffer and/or elution buffer and any other components desired as part of a process to purify nucleic acids from a sample.

The following examples illustrate specific embodiments of the invention described in this document. As would be apparent to skilled artisans, various changes and modifications are possible and are contemplated within the scope of the invention described.

### EXAMPLE 1

### Production of Hydrated Zirconium Silicate Composition

A four gram aliquot of zirconium silicate beads from Cole-Palmer was added to 80mL of water containing 5.32 grams sodium hydroxide (NaOH). The resulting suspension was refluxed for 72 hours, and then cooled to room temperature. The suspension was then rendered acidic (pH of 6.0 or lower) by addition of 100mL of 10% sulfuric acid (H₂SO₄). This acidic suspension was stirred at room temperature for one hour, and then filtered and washed three times with 50mL water and three times with 50mL acetone. Finally, the beads were oven dried for one hour at 100°C.

The resultant hydrated zirconium silicate beads were sufficiently electropositive to bind nucleic acids which could then be easily eluted therefrom as shown in the subsequent Examples.

### EXAMPLE 2

### Binding and Elution of Nucleic Acid To and From Hydrated Zirconium Silicate Composition

This experiment was performed to determine how the synthesis protocol for hydration of zirconium silicate beads effects the product's ability to bind/elute nucleic acid. Each of the three compositions (080596a, 080596b and 080596c)

The compositions of Example 1 were mixed with 200UL water followed by the addition of 1UL of 10⁶ P³² labeled M. tuberculosis DNA. The resulting suspension was incubated at room temperature 30 minutes on a rotator device. Following centrifugation the supernate was removed and added to 10mL of scintillation fluid for counting. 200UL of water was added to the remaining pellet and the resulting mixture was incubated at 65°C for 30 minutes. Following centrifugation the supernate was counted and the elution step repeated.

The results for the three lots of beads are shown below.

| **REACTION NO.** | **COMPOSITION USED******* | **mg OF COMPOSITION** | **CPM SUPER BINDING** | **CPM SUPER ELUTION #1** | **% DNA BOUND** | **% DNA ELUTED** |
|---|---|---|---|---|---|---|
| 1 | 080596a | 20 | 9031 | 1046 | 87 | 1 |
| 2 | | 10 | 34389 | 3648 | 52 | 10 |
| 3 | | 5 | 47895 | 7579 | 34 | 30 |
| 4 | | 2.5 | 52145 | 4357 | 28 | 21 |
| 5 | 080596b | 20 | 234 | 1442 | 100 | 2 |
| 6 | | 10 | 554 | 642 | 100 | 1 |
| 7 | | 5 | 469 | 681 | 100 | 1 |
| 8 | | 2.5 | 7333 | 3601 | 90 | 6 |
| 9 | 080596c | 20 | 1914 | 1469 | 97 | 2 |
| 10 | | 10 | 11311 | 4165 | 84 | 7 |
| 11 | | 5 | 38210 | 4437 | 47 | 13 |
| 12 | | 2.5 | 49782 | 2705 | 31 | 12 |

| **CONTROL NO SURFACE ADDED** | | | | | | |
|---|---|---|---|---|---|---|
| 13 | | | 72632 | | | |

| | | | | | | |
|---|---|---|---|---|---|---|
| *Lot 080596a 3X scale up that fit deeper into its heating mantel so that the reaction temperature was higher than Lot 080596b. Lot 080596b 3X scale up that was in a shallow heating mantel so it was cooler than Lot 080596a. Lot 080596c 1X reaction. | | | | | | |

### Conclusions

There were differences between the lots of hydrated zirconium silicate beads in terms of their ability to bind and elute DNA. Lot 080596b bound 100% of 10⁶ targets using only 5mg of the composition. This was unexpected given the cooler reaction temperature which would have suggested that this lot would be less reactive than Lot 080596a with DNA.

Also, Lot 080596b appears to form the tightest interaction with the DNA as it retains a higher percentage of DNA for all levels of composition tested than the other two compositions tested.

There is a trend that as the weight of composition in the reaction is reduced, there is less DNA bound but more DNA eluted for all surfaces tested. This indicates that there may be some type of equilibrium between bound and eluted DNA in solution that depends on the amount of hydrated composition present.

### EXAMPLE 3

### Increasing pH Effect or Elution of Nucleic Acid from Hydrated Zirconium Silicate Compositions

This experiment was performed to determine if nucleic bound to hydrated zirconium silicate beads (Lot 080596b of Example 2) would elute at various pHs.

The same protocol as used in Example 2 was used to bind M. tuberculosis DNA to 10mg of beads of Lot 080596b, except that following the first centrifugation, in addition to water, different concentrations of NaOH were used as elution buffers. The results are set forth below.

| **REACTION NO.** | **ELUTION BUFFER** | **CPM SUPER BINDING** | **CPM SUPER ELUTION #1** | **CPM SUPER ELUTION #2** | **% DNA BOUND** | **% DNA ELUTED** |
|---|---|---|---|---|---|---|
| 1 | Water | 461 | 542 | 234 | 100 | 1 |
| 2 | NaOH pH10 | 422 | 55140 | 9767 | 100 | 93 |
| 3 | NaOH pH12 | 369 | 70322 | 3096 | 100 | 100 |
| 4 | NaOH 1N | 303 | 71518 | 3755 | 100 | 100 |

| **CONTROL NO SURFACE ADDED** | | | | | | |
|---|---|---|---|---|---|---|
| 5 | | 69619 | | | | |

### Conclusions

There does seem to be a significant correlation between pH of the elution buffer and percentage DNA eluted, although other factors may be involved.

### EXAMPLE 4

### Effects of Elution Buffers on Nucleic Acid Bound to Hydrated Zirconium Silicate Compositions

This experiment was performed to determine if nucleic acid bound to hydrated zirconium silicate beads (Lot 080596b) would elute in various buffers.

200 uL water was added to a centrifuge tube containing 10mg of hydrated zirconium silicate beads followed by 1uL of DNA (10⁶ copies of M. tuberculosis genomic DNA). The resulting suspension was incubated at room temperature on a rotator device for 30 minutes. Following centrifugation, the supernate was removed and added to 10mL scintillation fluid and counted. 200uL of buffer was added to the pellet. The resulting suspension was incubated at 65°C for 30 minutes. Following centrifugation the supernate was removed and counted as above and the elution step repeated. The results are shown below.

| **REACTION NO.** | **ELUTION BUFFER** | **CPM SUPER BINDING** | **CPM SUPER ELUTION #1** | **CPM SUPER ELUTION #2** | **% DNA BOUND** | **% DNA ELUTED** |
|---|---|---|---|---|---|---|
| 1 | Water | 565 | 668 | 266 | 100 | 1 |
| 2 | NaOH pH7.2 | 573 | 4053 | 5254 | 100 | 11 |
| 3 | NaHCO3 pH9.4 | 579 | 61630 | 3996 | 100 | 81 |
| 4 | 25MMKPI pH7.6 | 414 | 63961 | 5055 | 100 | 85 |
| 5 | KPDG pH7.6 | 475 | 64701 | 2564 | 100 | 83 |

| **CONTROL NO SURFACE ADDED** | | | | | | |
|---|---|---|---|---|---|---|
| 6 | | 81466 | | | | |

### Conclusions

The buffers used elute much more DNA than does water.

### EXAMPLE 5

### Nucleic Acid Binding Conditions for Hydrated Zirconium Silicate Compositions

This experiment was performed to determine preferred binding conditions for nucleic acid to 5mg of hydrated zirconium silicate composition (Lot #080596b).

The same protocol as used in Example 2 was followed except that binding buffers and incubation times were used as shown below, and elution centrifugations were not performed. The results are shown below.

| **REACTION NO.** | **BINDING BUFFER** | **BINDING TEMP C** | **CPM SUPER BINDING** | **% DNA BOUND** |
|---|---|---|---|---|
| 1 | H20 | 4 | 630 | 97 |
| 2 | H20 | 25 | 506 | 98 |
| 3 | H20 | 37 | 1098 | 96 |
| 4 | 25MM KPI pH 7.6 | 4 | 21352 | 0 |
| 5 | 25MM KPI pH 7.6 | 25 | 22045 | 0 |
| 6 | 25MM KPI pH 7.6 | 37 | 20302 | 0 |
| 7 | HCl pH 4 | 4 | 70 | 100 |
| 8 | HCl pH 4 | 25 | 543 | 98 |
| 9 | HCl pH 4 | 37 | 401 | 98 |
| 10 | NaOH pH 10 | 4 | 22941 | 0 |
| 11 | NaOH pH 10 | 25 | 19875 | 1 |
| 12 | NaOH pH 10 | 37 | 20681 | 0 |

| **CONTROL NO SURFACE ADDED** | | | | |
|---|---|---|---|---|
| 13 | | | 20802 | |

### Conclusions

Water and HCl used in this experiment are both acidic and gave better binding of nucleic acid to hydrated zirconium silicate composition as compared to the other binding buffers, both of which are basic.

Temperature, at least under conditions of this experiment, did not seem to affect binding.

### EXAMPLE 6

### Additional Nucleic Acid Binding Conditions for Hydrated Zirconium Silicate Compositions

This experiment was performed to further evaluate binding conditions for hydrated zirconium silicate beads. In this experiment volume, time and whether or not to use the rotator device for the binding step were evaluated.

5mg of hydrated zirconium silicate beads (Lot 080596b) were added to water at one of three volumes. 1ul of ³²P labeled M. tuberculosis DNA 10⁶ targets were added. The resulting suspension was either incubated on the rotator device or incubated without rotating for 1 of 3 times. Following centrifugation the supernate was removed and added to 10mL scintillation fluid for counting. The results are set forth below.

| **REACTION NO.** | **VOLUME OF BINDING BUFFER uL** | **LENGTH OF BINDING STEP MIN.** | **ROTATED OR NOT** | **CPM SUPER BINDING** | **% DNA BOUND** |
|---|---|---|---|---|---|
| 1 | 50 | 10 | N | 3076 | 85 |
| 2 | 50 | 30 | N | 761 | 96 |
| 3 | 50 | 50 | N | 392 | 98 |
| 4 | 50 | 10 | Y | 132 | 100 |
| 5 | 50 | 30 | Y | 131 | 100 |
| 6 | 50 | 50 | Y | 102 | 100 |
| 7 | 200 | 10 | N | 7196 | 65 |
| 8 | 200 | 30 | N | 2269 | 89 |
| 9 | 200 | 50 | N | 372 | 98 |
| 10 | 200 | 10 | Y | 252 | 99 |
| 11 | 200 | 30 | Y | 268 | 99 |
| 12 | 200 | 50 | Y | 132 | 100 |
| 13 | 500 | 10 | N | 5059 | 76 |
| 14 | 500 | 30 | N | 1398 | 93 |
| 15 | 500 | 50 | N | 4164 | 80 |
| 16 | 500 | 10 | Y | 4592 | 78 |
| 17 | 500 | 30 | Y | 2369 | 89 |
| 18 | 500 | 50 | Y | 1395 | 93 |

| **CONTROL NO SURFACE ADDED** | | | | | |
|---|---|---|---|---|---|
| 19 | | | | 20767 | |

### Conclusion

Smaller volumes of binding buffer and longer incubation times appear to allow for a higher percentage DNA bound to the beads. Also, use of the rotator device helps to reduce the incubation time necessary for near 100% binding of the DNA.

### EXAMPLE 7

### Effect of Contaminants on Binding of Target Nucleic Acid by Hydrated Zirconium Silicate Composition

This experiment was performed to determine the effect of human DNA and/or BSA on the percent of target nucleic acid (M. tuberculosis) bound to hydrated zirconium silicate beads (Lot 080596b).

5mg of hydrated zirconium silicate beads were added to 200ul water followed by the addition of human DNA and/or BSA. Next ³²P labeled M. tuberculosis DNA (1uL) containing 10⁶ genomic copies was added. The resulting suspension was incubated on a rotator device for 30 minutes. Following centrifugation, the supernate was removed and added to 10mL scintillation fluid for counting.

| **REACTION NO.** | **HUMAN DNA uL******* | **BSA uL******* | **CPM SUPER BINDING** | **% DNA BOUND** |
|---|---|---|---|---|
| 1 | 0 | 0 | 1640 | 92 |
| 2 | 1 | 0 | 1296 | 93 |
| 3 | 2 | 0 | 5756 | 71 |
| 4 | 3 | 0 | 5752 | 71 |
| 5 | 4 | 0 | 5187 | 71 |
| 6 | 5 | 0 | 5511 | 71 |
| 7 | 10 | 0 | 11455 | 42 |
| 8 | 0 | 1 | 144 | 100 |
| 9 | 0 | 5 | 1636 | 92 |
| 10 | 0 | 10 | 1187 | 92 |
| 11 | 1 | 1 | 4621 | 77 |
| 12 | 5 | 5 | 7927 | 60 |
| 13 | 10 | 10 | 13417 | 33 |

| **CONTROL NO SURFACE ADDED** | | | | |
|---|---|---|---|---|
| 14 | | | 19880 | |

| | | | | |
|---|---|---|---|---|
| * BSA and human DNA solutions are both 1uG/uL | | | | |

### Conclusions

Control reaction #1 normally binds 98 - 100% of the M. tuberculosis DNA but is only 92% for this experiment. If this is assumed to be an error, then it can be assumed that the presence of 1ug of human DNA has a significant effect on the percentage of target DNA bound. However, without that assumption there is a clear effect when 2ug of human DNA is added. The same effect is seen when the amount of human DNA is increased from 2 to 5ug but the addition of 10ug cuts the amount of M. tuberculosis bound to 42%. This effect may be reduced by using more of the composition or, in a clinical setting, by using a pre-filter to remove human DNA prior to release of target nucleic acid from the cells of the organism.

The presence of BSA has a much less significant effect on M. tuberculosis DNA binding. 5 or 10ug of BSA appears to reduce the binding of M. tuberculosis DNA by about 8-11%, either with or without human DNA.

### EXAMPLE 8

### Decreasing pH Effect on Elution of Nucleic Acid from Hydrated Zirconium Silicate Composition

This experiment was performed to determine if lowering the pH of the elution buffer will effect the efficiency of DNA elution for compositions of the present invention. Previously, it was shown that raising the pH had a positive effect on DNA elution from these surfaces.

Two acids were tested at two different pH values each. Specifically, 5mg of each surface was added to 200uL of water followed by 1uL of ³²P labeled DNA (10⁶ genomic copies of M. tuberculosis DNA). The resulting suspension was incubated on a rotator device for 30 minutes. Following centrifugation, the supernate was removed and added to 10mL scintillation fluid for counting. 200uL of the elution buffer was added to the remaining pellet and the resulting suspension was incubated at 65°C for 30 minutes. Following centrifugation the supernate was removed and counted as above. The results are shown below.

| **REACTION No.** | **SURFACE** | **ELUTION BUFFER** | **CPM SUPER BINDING** | **% DNA BOUND** | **CPM SUPER ELUTION** |
|---|---|---|---|---|---|
| 1 | 080596b | H20 | 1581 | 90 | 730 |
| 2 | | HCl pH 4 | 134 | 100 | 1173 |
| 3 | | HCl pH 2 | 371 | 98 | 184 |
| 4 | | H3PO4 pH 4 | 319 | 98 | 830 |
| 5 | | H3PO4 pH 2 | 439 | 97 | 1002 |
| 6 | 7-17-96 (SFSF)b | H20 | 515 | 96 | 1462 |
| 7 | | HCl pH 4 | 626 | 95 | 697 |
| 8 | | HCl pH 2 | 351 | 98 | 936 |
| 9 | | H3PO4 pH 4 | 295 | 98 | 1353 |
| 10 | | H3PO4 pH 2 | 515 | 96 | 788 |
| 11 | 7-8-96545a | H20 | 2489 | 86 | 2154 |
| 12 | | HCl pH 4 | 2322 | 85 | 1189 |
| 13 | | HCl pH 2 | 2547 | 87 | 1139 |
| 14 | | H3PO4 pH 4 | 2961 | 83 | 1784 |
| 15 | | H3PO4 pH 2 | 2912 | 83 | 1183 |

| **CONTROL NO SURFACE ADDED** | | | | | |
|---|---|---|---|---|---|
| 16 | | | 16906 | | |

### Conclusions

Lowering the pH appears not to affect the amount of M. tuberculosis eluted compared to water. It may, however, be useful for getting DNA to bind to the composition. Also, binding of DNA to the compositions is very reproducible.

### EXAMPLE 9

### Comparison of Hydrated Zirconium Silicate Composition to Hydrated Celite Composition

This experiment was performed to compare the compositions of the present invention to hydrated Celite and to determine if ionic strength and pH of the elution buffer effects DNA elution from hydrated compositions. Two surfaces were evaluated in this experiment; hydrated zirconium silicate beads (Lot 080596b) and hydrated Celite 545 (Lot 7-8-96545b). Also, the effect of temperature on DNA elution was evaluated.

10 mg of each composition were combined with 200uL H₂0, followed by 1uL of M. tuberculosis DNA 10⁶ copies, P³² labeled. The resulting suspension was incubated at room temperature on a rotator device for 30 minutes. Following centrifugation the supernate was removed and added to 10mL scintillation fluid for counting. To the remaining pellet was added 200uL of elution buffer. The resulting suspension was incubated at 65°C or room temperature for 30 minutes. Following centrifugation the supernate was removed and counted as in Examples above. The results are shown below.

| **REACTION NO.** | **SURFACE** | **ELUTION BUFFER** | **ELUTION TEMP.** | **CPM SUPER BINDING STEP** | **CPM SUPER ELUTION** | **% DNA ELUTED** |
|---|---|---|---|---|---|---|
| 1 | 080596b | H20 | 55C | 434 | 673 | 1 |
| 2 | | NaOH pH10 | RT | 696 | 37422 | 67 |
| 3 | | NaOH pH12 | RT | 424 | 45936 | 82 |
| 4 | | NaOH 1N | RT | 360 | 43375 | 77 |
| 5 | | 25MM NaCl | 55C | 435 | 306 | 0 |
| 6 | | 250MM NaCl | 55C | 318 | 257 | 0 |
| 7 | | 25MM KCl | 55C | 384 | 325 | 0 |
| 8 | | 250MM KCl | 55C | 213 | 647 | 1 |
| 9 | 7-8-96545b | H20 | 55C | 11038 | 4150 | 9 |
| 10 | | NaOH pH10 | 55C | 10892 | 7082 | 16 |
| 11 | | NaOH pH12 | 55C | 11802 | 41913 | 93 |
| 12 | | NaOH 1N | 55C | 10972 | 41775 | 93 |

| **CONTROL NO SURFACE ADDED** | | | | | | |
|---|---|---|---|---|---|---|
| 13 | | | | 56151 | | |

### Conclusions

Ionic strength appears to have little effect on the elution of DNA from hydrated surfaces whereas pH appears to be the more important factor in DNA elution. Also, compared with earlier experiments, the elution of DNA is more efficient at higher temperatures for the zirconium silicate beads and probably would be for the 545 Celite as well. Finally, under identical conditions, the hydrated zirconium silicate composition of the present invention binds significantly more DNA than the hydrated Celite 545.

### EXAMPLE 10

### Preferred Buffer Volume and Time for Elution of Bound Nucleic Acid from Hydrated Zirconium Silicate Compositions

This experiment was performed to determine preferred elution conditions for hydrated zirconium silicate compositions of the present invention.

5mg of hydrated zirconium silicate beads (Lot 080596b) was added to 200uL of water followed by 1uL containing 10⁶ genomic copies of ³²P labeled M. tuberculosis DNA. The resulting suspension was incubated on a rotator device for 30 minutes. Following centrifugation the supernate was removed and added to 10mL scintillation fluid for counting. Elution buffer (25mm KPI pH 7.6) was added at one of three volumes below. The resulting suspension was incubated at 55°C for 10, 30, or 50 minutes. Following centrifugation the supernate was removed and counted as above. The results are shown below.

| **REACTION NO.** | **VOLUME ELUTION BUFFER** | **TIME OF ELUTION** | **CPM SUPER BINDING** | **CPM SUPER ELUTION** | **% DNA BOUND** | **% DNA ELUTED** |
|---|---|---|---|---|---|---|
| 1 | 100 | 10 | 436 | 14534 | 98 | 76 |
| 2 | 100 | 30 | 515 | 17349 | 98 | 91 |
| 3 | 100 | 50 | 274 | 22277 | 99 | 100 |
| 4 | 200 | 10 | 179 | 17313 | 99 | 91 |
| 5 | 200 | 30 | 250 | 18010 | 99 | 95 |
| 6 | 200 | 50 | 294 | 18535 | 99 | 96 |
| 7 | 500 | 10 | 176 | 20370 | 99 | 100 |
| 8 | 500 | 30 | 265 | 18072 | 99 | 95 |
| 9 | 500 | 50 | 129 | 18845 | 99 | 97 |

| **CONTROL NO SURFACE ADDED** | | | | | | |
|---|---|---|---|---|---|---|
| 10 | | | 19587 | 19587 | | |

### Conclusions

Using 500mL of elution buffer, 100% of the DNA is eluted after only 10 minutes and for that elution volume, longer elution times actually reduce the amount of DNA eluting. Using 200uL or 100uL elution buffer volumes, the %DNA eluted increased each time the elution incubation time was increased.

### EXAMPLE 11

### Preferred Temperature and Ionic Strength of KPI Buffer for Elution of Bound Nucleic Acid from Hydrated Zirconium Silicate Compositions

This experiment was performed to determine preferred elution conditions of ³²P labeled nucleic acid bound to hydrated zirconium silicate beads (Lot 080596b).

5mg of the hydrated zirconium silicate beads were added to 200uL of water followed by 1uL containing 10⁶ M. tuberculosis genomic copies. The resulting suspension was incubated on a rotator device for 30 minutes. Following centrifugation, the supernate was removed and added to 10ml of scintillation fluid for counting. 200uL of elution buffer was added and the resulting suspension was incubated at one of four temperatures for 30 minutes. The incubation was done either with or without the rotator device used in the binding step. Following centrifugation the supernate was removed and counted as above. The results are shown below.

| **REACTION NO.** | **ELUTION BUFFER** | **ELUTION TEMP** | **ROTATED** | **CPM SUPER BINDING** | **CPM SUPER ELUTION** | **% DNA BOUND** | **% DNA ELUTE D** |
|---|---|---|---|---|---|---|---|
| 1 | 25MM KPI pH7.6 | RT | N | 977 | 12578 | 93 | 100 |
| 2 | | 37 | N | 268 | 14250 | 98 | 100 |
| 3 | | 55 | N | 284 | 14633 | 98 | 100 |
| 4 | | 65 | N | 272 | 13833 | 98 | 100 |
| 5 | | RT | Y | 322 | 10423 | 98 | 78 |
| 6 | 500MM KPI pH7.6 | RT | N | 285 | 13344 | 98 | 100 |
| 7 | | 37 | N | 203 | 12394 | 98 | 92 |
| 8 | | 55 | N | 335 | 14061 | 98 | 100 |
| 9 | | 65 | N | 223 | 14967 | 98 | 100 |
| 10 | | RT | Y | 282 | 12634 | 98 | 94 |

| **CONTROL NO SURFACE ADDED** | | | | | | | |
|---|---|---|---|---|---|---|---|
| 11 | | | | 13760 | | | |

### Conclusions

There does not appear to be an advantage to using 500MM KPI compared to 25MM KPI. Either buffer gives near 100% elution of the DNA under almost any conditions tested. Increased temperature of the elution reaction does not appear to have an effect on the elution process. Using the rotator device gave less than 100% DNA eluted in both buffers indicating that it is not needed for the elution process.

### EXAMPLE 12

### Wash Conditions for Hydrated Zirconium Silicate Compositions

This experiment was performed to determine preferred wash conditions for ³²P labeled nucleic acid bound to hydrated zirconium silicate beads (Lot 080596b). The wash buffer was either stored at 4°C or at room temperature for both buffers tested.

5mg of the hydrated zirconium silicate composition was added to 200uL of water followed by 1uL containing 10⁶ ³²P labeled M. tuberculosis genomic copies. The resulting suspension was incubated on a rotator device for 30 minutes. Following centrifugation the supernate was removed and added to 10mL scintillation fluid for counting. 200uL of the wash buffer was added to the remaining pellet. The resulting suspension was vortexed approximately 10 seconds and quickly centrifuged. The supernate was removed and counted as above. The wash step was either repeated or not repeated. 200uL of 25MM KPI pH7.6 was added and the resulting suspension was incubated at 65°C for 30 minutes. Following centrifugation the supernate was counted as above. The results are shown below.

| **REACTION NO.** | **WASH BUFFER** | **WASH BUFF TEMP** | **WASHES** | **CPM SUPER BINDING** | **CPM SUPER WASH #1** | **CPM SUPER WASH #2** | **CPM SUPER ELUTION** |
|---|---|---|---|---|---|---|---|
| 1 | Water | 4 | 1 | 334 | 341 | | 12921 |
| 2 | | 4 | 2 | 2083 | 301 | 329 | 11402 |
| 3 | | RT | 1 | 1644 | 273 | | 11629 |
| 4 | | RT | 2 | 711 | 236 | 274 | 12679 |
| 5 | 25mmKP I | 4 | 1 | 358 | 1487 | | 11991 |
| 6 | | 4 | 2 | 291 | 1791 | 1491 | 10479 |
| 7 | | RT | 1 | 262 | 3477 | | 9077 |
| 8 | | RT | 2 | 335 | 3442 | 3000 | 7728 |

| **CONTROL NO SURFACE ADDED** | | | | | | | |
|---|---|---|---|---|---|---|---|
| 9 | | | | 13964 | | | |

### Conclusions

Using water as the wash buffer, the temperature of the wash solution does not seem to effect the amount of DNA that elutes during the wash step. Also, doing two washes does not significantly effect the amount of DNA eluted.

However, using 25MM KPI as the wash buffer has a more dramatic effect on DNA elution during the wash step. At 4°C ther elution is moderate, but more extensive than when water is used. When it was at room temperature approximately 25% of the DNA is lost during each wash step.

### EXAMPLE 13

### Determination of Physical Characteristics of Suspension of Hydrated Zirconium Silicate Composition in Water

This experiment was performed to measure various physical characteristics of a suspension containing a composition of the present invention in water. The results will be compared to the nucleic acid binding/elution data obtained earlier to attempt to predict good or bad binding characteristics of different lots of binding compositions.

125mg of the hydrated zirconium silicate composition of the present invention was added to 3mL water and the pH measured. One more mL of water was added and the conductivity and the osmotic pressure was measured. For osmotic pressure readings, 10uL of the sample was used. The results are set forth below.

| **SURFACE LOT NO.** | **% DNA BOUND IN WATER** | **% DNA ELUTED** | **pH** | **CONDUCTIVITY** | **OSMOTIC PRESSURE** |
|---|---|---|---|---|---|
| 080596b | 100 | 100 | 4.82 | 335us | 116 |
| 081996b | 99 | 94 | 4.55 | 210us | 110 |
| 080596a | 40 | 100 | 4.81 | 294us | 113 |
| 081996a | 98 | 90 | 4.40 | 1326us | 112 |
| 081696a | 20 | 75 | 10.30 | 683us | 113 |
| 081496b | 0 | 0 | 11.36 | 2.89Kus | 147 |
| 7-17-96sfsfb | 99 | 97 | 4.69 | 3.25Kus | 114 |
| 7-8-96545b | 92 | 100 | 1.49 | 11.52Kus | 136 |
| 7-8-96545a | 93 | 100 | 2.30 | 2.63Kus | 115 |
| 7-29-96bsolid | 92 | 100 | 4.46 | 685us | 110 |
| 7-29-96c | 2 | 0 | 9.39 | 510us | 122 |
| 082396a | 50 | 98 | 7.91 | 930us | 122 |
| BLANK WATER | | | 6.84 | 344us | 118 |

### Conclusions

Compositions that decrease the pH of water from 6.84 are good nucleic acid binders except for the hydrated zirconium silicate composition 080596a. Compositions that increase the pH of water are poor DNA binders. These results are consistent with those previously seen in other Examples herein for nucleic acid binding buffer analysis that showed buffers below the pH of water were good binders and poor eluters but that buffers with a pH higher than water were poor binding buffers but good elution buffers (see Examples 4, 5, 8 and 9).

### EXAMPLE 14

### Determination of Physical Characteristics of Suspension of Hydrated Zirconium Silicate Compositions in KPI

This experiment was performed to measure various physical characteristics of a suspension containing a composition of the present invention in 25MM KPI. The results will be compared to the nucleic acid binding/elution data obtained earlier to attempt to predict good or bad lots of nucleic acid binding compositions.

125 mg of the hydrated zirconium silicate composition of the present invention was added to 3mL KPI buffer (25MM KPI, pH 7.6) and the pH measured. One more mL of buffer was added and the conductivity and the osmotic pressure was measured. For osmotic pressure readings, 10uL of the sample was used. The results are shown below.

| **SURFACE LOT NO.** | **% DNA BOUND IN WATER** | **% DNA ELUTED IN 25MM KPI, pH 7.6** | **pH** | **CONDUCTIVITY Kus** | **OSMOTIC PRESSURE** |
|---|---|---|---|---|---|
| 080596b | 100 | 100 | 7.63 | 4.57 | 183 |
| 081996b | 99 | 94 | 7.63 | 4.14 | 188 |
| 080596a | 40 | 100 | 7.59 | 5.05 | 190 |
| 081996a | 98 | 90 | 7.45 | 4.95 | 196 |
| 081696a | 20 | 75 | 9.15 | 4.03 | 194 |
| 081496b | 0 | 0 | 10.85 | 6.49 | 216 |
| 7-17-96sfsfb | 99 | 97 | 6.95 | 6.45 | 215 |
| 7-8-96545b | 92 | 100 | 2.11 | 8.87 | 220 |
| 7-8-96545a | 93 | 100 | 6.57 | 4.84 | 204 |
| 7-29-96bsolid | 92 | 100 | na | na | na |
| 7-29-96c | 2 | 0 | 7.80 | 3.89 | 194 |
| 082396a | 50 | 98 | 7.61 | 3.99 | 200 |
| BLANK WATER | | | 7.65 | 3.80 | 198 |

### Conclusions

Compositions do not necessarily increase or decrease the pH of the KPI buffer, but if the composition does lower the pH from 7.65 it was a good nucleic acid binder, and if it raised the pH of the KPI buffer it was a poor nucleic acid binder.

The zirconium silicate composition 080596b binds DNA very well in water but it binds no DNA in 25MMKPI pH7.6. In water, the pH of the solution with 080596b in it is 4.82, and in 25MMKPI it is 7.63. This difference in pH is probably the reason for the differences in DNA binding in these two buffers. It is possible that one of the compositions that lowers the pH of the KPI to acidic range would be a good DNA binder in 25MMKPI.

### EXAMPLE 15

### Amplification of Nucleic Acid Bound by Hydrated Zirconium Silicate Compositions

This experiment was performed to determine if nucleic acid bound and eluted from various hydrated compositions will amplify and to determine if the compositions will partially dissolve in the elution buffer and thereby inhibit a Strand Displacement Amplification reaction.

3mg of each composition below was added to 200uL of H₂0 followed by 1uL containing 10⁶ M. tuberculosis DNA targets. The resulting suspension was incubated on a rotator device for 30 minutes. Following centrifugation, the supernate was removed and discarded. 30uL of 25MM KPI was added to the remaining pellet. The resulting suspension was incubated at 65°C for 30 minutes. Following centrifugation, the supernate was removed and 25uL was run in thermophilic SDA reactions as follows. A master mix of thermophilic tSDA reagents was prepared containing the following reagents:

| **REAGENTS** | **CONCENTRATION** | **AMT/RXN** |
|---|---|---|
| KPO4 | 500 mM | 1.5 |
| MgOAc | 600 mM | 0.5 |
| Glycerol | 50% | 4 |
| DMSO | 50% | 5 |
| BSA | 5mg/ml | 1 |
| dNTP's | 20X | 2.5 |
| P+B | 20X | 2.5 |
| HDNA | 200ng/ul | 2.5 |
| Water | | 1.5 |

A master mix of tSDA enzymes was also prepared containing:

| **REAGENTS** | **CONCENTRATION** | **AMT/RXN** |
|---|---|---|
| BsoBl | 160 Units | 2.25 |
| Bst | 20 Units | 0.8 |
| Glycerol | 50% | 0.95 |

The reagent master mix was aliquoted to individual tubes in an amount of 21uL, and 25uL of elution buffer containing target eluted from the hydrated zirconium silicate beads and comparative Celite materials was added to each tube. The tubes were heated to 95°C for 3 minutes to denature any target, and then placed in a heating block at 52.5°C for 3 minutes. A 4uL aliquot of the enzyme master mix was added to the tubes which were then incubated at 52.5°C for 30 minutes. The tubes were then heated to 95°C for 3 minutes to inactivate the enzymes.

Using labeled probes for the target M. tuberculosis sequence in a chemiluminescence detection assay, the reaction products were assayed for relative light units (RLU) which indicate amplification of target nucleic acid. For comparison, corresponding blank tubes without any target DNA and with spiked target DNA (100% recovery of target) were run in parallel. The results are shown below.

| **REACTION NO.** | **SURFACE** | **RLU DNA BOUND AND ELUTED** | **RLU DNA ADDED AFTER ELUTION** | **RLU NO DNA** |
|---|---|---|---|---|
| 1 | 080596b | 26214 | 35158 | 16 |
| 2 | | 8102 | 21089 | 15 |
| 3 | | 23738 | 27458 | 16 |
| 4 | 7-17-96(SFSF)b | 22371 | 15492 | 12 |
| 5 | | 27547 | 29202 | 20 |
| 6 | | 15493 | 13641 | 17 |
| 7 | 7-8-96545a | 18347 | 32215 | 12 |
| 8 | | 10207 | 25541 | 15 |
| 9 | | 9373 | 19740 | 25 |

| CONTROL NO SURFACE ADDED | | | | |
|---|---|---|---|---|
| 10 | | 32090 | | 21 |
| 11 | | 28532 | | 19 |
| 12 | | 24926 | | 15 |

### Conclusions

The SDA signals for amplification of DNA from the hydrated zirconium silicate beads and the hydrated SFSF Celite are somewhat higher than the signals generated using hydrated Celite 545. However, none of the surfaces are as high as the control signal and there are some wide ranges in signals within a surface's triplicate runs. This may indicate that there was some DNA not delivered to the tSDA reaction and/or the amount varies slightly from reaction to reaction. Optimization of the reaction would rectify these inconsistencies.

While the invention has been described with some specificity, modifications apparent to those with ordinary skill in the art may be made without departing from the scope of the invention. Various features of the invention are set forth in the following claims.

## Claims

1. A hydrated zirconium silicate composition which binds nucleic acid.

2. The hydrated zirconium silicate composition of claim 1 which binds nucleic acid in water.

3. The hydrated zirconium silicate composition of claim 1 having a density of at least 2.0mg/uL.

4. The hydrated zirconium silicate composition of claim 1 which in an amount of 2.5 milligrams will bind at least about 85 percent of 10⁶ copies of a target nucleic acid in a sample without other nucleic acids.

5. A process for purifying nucleic acid from a sample comprising the steps of:
(a) exposing the sample to a hydrated zirconium silicate composition for a period of time sufficient for nucleic acid in the sample to bind to said composition; and
(b) separating said composition from the sample.

6. The process of claim 5 further comprising:
(c) recovering nucleic acid from said composition.

7. A kit for purifying nucleic acid from a sample comprising the hydrated zirconium silicate composition of claim 1.

8. The kit of claim 7 wherein said hydrated zirconium silicate composition is present as particles.

9. The kit of claim 8 wherein said particles are beads.

10. A process for binding and subsequently eluting nucleic acid from a surface capable of binding said nucleic acid comprising the steps of:
(a) contacting said nucleic acid with said surface in the presence of a buffer solution with a pH below about 7.0; and
(b) eluting said nucleic acid from said surface in the presence of a buffer solution with a pH above about 7.0.
